(19) Europäisches Patentamt
European Patent Office
Office européen des brevets

(11) Publication number: 0 001 133
B1

(12) EUROPEAN PATENT SPECIFICATION

(45) Date of publication of patent specification: 20.01.82

(21) Application number: 78200140.8

(22) Date of filing: 11.08.78

(51) Int. Cl.[3]: C 07 D 499/12, C 07 D 499/68 //C07C101/20

# (54) Process for the preparation of 6-[D-alpha-amino-(p-hydroxyphenyl)-acetamido]penicillanic acid.

(30) Priority: 06.09.77 NL 7709812

(43) Date of publication of application:
21.03.79 Bulletin 79/6

(45) Publication of the grant of the European patent:
20.01.82 Bulletin 82/3

(84) Designated Contracting States:
BE CH DE FR GB LU NL SE

(56) References cited:
DE - A - 2 418 719
DE - A - 2 450 661
DE - A - 2 454 841
GB - A - 1 400 236
US - A - 3 912 719

CHEMICAL ABSTRACTS, vol. 84, no. 19, May 10, 1976, page 499; 135638u Aminopenicillins

CHEMICAL ABSTRACTS, vol. 85, no. 23, December 6, 1976, page 524; 177405s, Wide-spectrum semisynthetic penicillin.

(73) Proprietor: Gist - Brocades N.V.
Wateringseweg 1 P.O. Box 1
NL-2600 MA Delft (NL)

(72) Inventor: Henniger, Peter Wolfgang
Lammenschansweg 65
NL-2313 DK Leiden (NL)
Inventor: Van Der Drift, Johannes Karel
Marterstraat 39
NL-2623 ED Delft (NL)
Inventor: Van Veen, Gerard Joannes
Thorbeckelaan 102
NL-2641 XJ Pijnacker (NL)

(74) Representative: Mars, Pieter et al,
c/o Gist-Brocades N.V. Patents and Trademarks
P.O.Box 1
NL-2600 MA Delft (NL)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

Process for the preparation of 6-[D-α-amino-(p-hydroxyphenyl)acetamido]penicillanic acid

The invention relates to a process for the preparation of 6-[D-α-amino-(p-hydroxyphenyl)-acetamido]penicillanic acid (commonly named amoxicillin) and non-toxic pharmaceutically acceptable salts thereof by the acylation of derivatives of 6-aminopenicillanic acid with an acylating agent derived from p-hydroxyphenylglycine, and relates more specifically to a process consisting of the acylation of a compound having the following formula

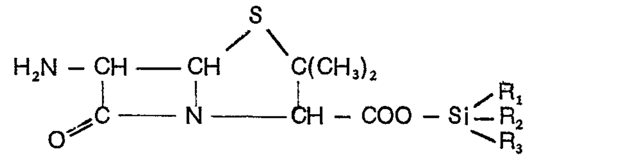

(I)

and/or

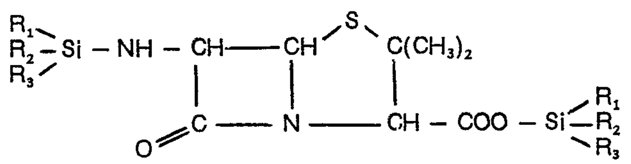

(II)

wherein each of $R_1$, $R_2$ and $R_3$ represents the same or different alkyl group of 1—3 carbon atoms, and each preferably represents a methyl group, with an acylating agent according to the general formula

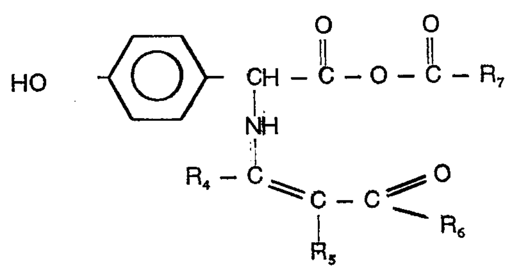

(III)

wherein $R_4$ represents an alkyl group of 1—3 carbon atoms and preferably a methyl group, $R_5$ represents a hydrogen atom or an alkyl group of 1—3 carbon atoms and preferably a methyl group, $R_6$ represents an alkoxy group of 1—3 carbon atoms and a preferably methoxy group and $R_7$ represents an alkoxy group of 1—2 carbon atoms and preferably a methoxy group.

Such a process for the preparation of 6-[D-α-amino-p-(hydroxyphenyl)acetamido]-penicillanic acid is generally known from e.g. British Patent Specification no. 1,339,605 disclosing indeed the preparation of this compound according to Examples 1—5, but in economically non-interesting yields (Example 1: 43% of a purity of 80%; Example 3: 20% of a purity of 16%; Example 4: 37% of a purity of 93%, while the final parts of Examples 2 and 5 only just show the above-mentioned compound to be present.

On the other hand, a process which comprises the acylation of a previously silylated cephalosporanic acid derivative with a mixed anhydride according to the general formula III, is generally known from e.g. the published Dutch application NL 6912811, the British patent specification no. 1073530 and more particularly from the British patent specification no. 1460914.

However, especially from e.g. Examples 3 and 13 of the latter specification, a person skilled in the art cannot derive with sufficient clarity, that an acylation of e.g. 7-amino-3-((1H)-1,2,3-triazol-5-yl)-thiomethyl-3-cephem-4-carboxylic acid, or its trimethylsilyl derivative, by means of e.g. ethoxycarbonyl D-α-(1-carbomethoxy-propen-2-yl)amino-p-hydroxyphenyl acetate, could be carried out with economically attractive yields. More particularly in example 13, page 21, the conversion of 0.02 mol of 7-amino-3-(1,2,3-triazole-5-yl)-thiomethyl-3-cephem-4-carboxylic acid, which has previously been converted with 0.06 mol trimethylchlorosilane in the presence of 0.06 mol of an about 2:1 mixture of

triethylamine and N,N-dimethylaniline, with a reaction-mixture prepared in the usual way in acetonitrile from 0.05 mol of the Dane salt and ethylchloroformate, seems to lead only to a rather unattractive final yield (see e.g. page 21 line 38 in connection with lines 5—10 of the same page).

Also other patent literature relating to the preparation of this type of cephalosporanic acid derivatives showed only economically unattractive processes, e.g. US patent B 516.047, example 1, column 7, line 44, example 5A, line 52 and example 6, US patent 3,946,003, example 1 (column 11, line 65) and example 3 (column 12, line 62).

Moreover, according to column 4, lines 1—5 and example 1, (column 10), it seems necessary to first silylate the phenolic p-hydroxy group in order to obtain practical results, which necessity, moreover, also appears from example 1 on pages 13 and 14 of the German application DT-2600880, the examples 1—5 of German application DT-2626280, US patent 3946003, example 1 and Belgian patent 840060, example 10.

Therefore, it was less attractive for people skilled in the art to develop the method described in e.g. the British specification 1.339.605 than to look for alternative acylation methods in the search for a new and improved method for the preparation of the desired compounds with an economically attractive yield and a relatively high purity. An additional modern requirement for a process to be used on a technical scale is, moreover, that inevitably occurring impurities can be removed in a cheap and simple manner so as to achieve the conditions of purity dictated by health authorities.

It is known from literature to prepare penicillanic acid and cephalosporanic acid derivatives by acylating 6-APA or 7-A(D)CA and their derivatives with the hydrochloride of 2-phenylglycinechloride and derivatives thereof having a substituted phenyl group, whereby the said acid chloride is obtained by reacting the substituted phenylglycine with reagents like phosphorus pentachloride, thionyl chloride and phosgene. Although improved processes for the preparation of D-(—)-2-(p-hydroxyphenyl)glycyl chloride hydrochloride and the crystalline hemidioxane solvate thereof are known from British Patent Specifications no. 1460915 and no. 1466637, the acylation of 6-APA or 7-A(D)CA or its 3-methyl modifications with the above-mentioned acylating agent did not lead to the desired result, mainly because either the product formed was too impure to make its purification to the required quality worthwhile, or the starting D-2-(p-hydroxyphenyl)glycyl chloride hydrochloride of the required quality (purity) is only available at economically unattractive prices, if at all. The occurring impurities found, if an acid chloride hydrochloride in economically necessary amounts of acceptable prices is used, appear to be in agreement with the indications about the accompanying impurities in the final products and the low yields of the rather similar acylation with the said D-(—)-2-(p-hydroxyphenyl)glycyl chloride hydrochloride of 7-amino-cephalosporanic acid derivatives according to British Patent Specification no. 1460916 (viz. Examples 2, 4 and 9, especially page 17, lines 32—34 and Example 8 referring to further purification of the desired product) and British Patent Specification no. 1460914 (viz. Examples 2, 5, 9 and 10). German Patent Application DT 2520647 in this connection on page 2, lines 1—9 also discloses that generally used acylating agents such as acid halides cannot be properly applied in the amoxicillin synthesis. It is true that a process for the preparation of amoxicillin trihydrate was also known from the published German patent application DT 2611286, comprising the acylation of previously silylated 6-APA, with D-(—)-2-p-(hydroxyphenyl)glycyl chloride hydrochloride and leading to yields which seem to approach to some extent at least present practical requirements.

However, for the preparation of this starting material according to e.g. British patent specifications no. 1460915 and no. 1466637 phosgene is applied in a relatively difficultly manageable process in which a solid is reacted with a gas. Such an application is extremely expensive in a large number of countries with very stringent safety regulations, if indeed it may be applied at all.

For the same reason the process described in British patent specifications no. 1268536 and no. 1341827, disclosing the preparation of 6-isocyanatopenicillanic acid and 7-isocyanato cephalosporanic acid derivatives from 6-APA and 7-A(D)CA esters with phosgene and its subsequent reaction to form penicillins or cephalosporins, is left out of consideration for the preparation of amoxicillin.

On the other hand, German patent application no. 2520647, for example, discloses a process for the preparation of $\alpha$-amino-acylpenicillanic acid derivatives and — inter alia — amoxicillin, in which

(i) 6-APA is contacted with an excess of a strong tertiary amine base (e.g. triethylamine) in an inert, water-insoluble, organic solvent (e.g. methylene chloride or chloroform), resulting in a solution of a salt of 6-APA with the base in said solvent,

(ii) the remaining strong tertiary amine base is neutralised in the solvent, e.g. by addition of N,N-dimethylacetamide hydrochloride,

(iii) the obtained neutralised solution is contacted with a solution of a mixed anhydride of a short chain alkoxyformic acid and an N-protected derivative of D-2-amino-p-hydroxyphenylacetic acid, in which the N-protecting group is labile for acid, in a water-insoluble, inert, organic solvent at a temperature of —50° to +30°C, preferably —30°C to 0°C, resulting in a solution of an N-protected amoxicillin derivative,

(iv) the solution so obtained is contacted with water and a strong acid (such as hydrochloric acid or p-toluene-sulphonic acid) at room temperature or cooled to, e.g. 0°C, in order to remove the acid-labile N-protecting group and

(v) the thus obtained amoxicillin is isolated from the aqueous system.

Less attractive features of this process are, inter alia that the process is carried out at low concentrations, that solvents become mixed so that recovery thereof becomes more difficult and that, when adding the N,N-dimethylacetamide hydrochloride, due to local high concentrations, 6-APA sometimes crystallizes, so that a very accurate dosing scheme is required.

Furthermore, a number of patent specifications disclose preparation methods of $\alpha$-aminoacyl penicillanic acid derivatives by acylating 6-APA with mixed anhydrides derived from modified Dane salts of D-2-amino-(p-hydroxyphenyl)acetic acid, e.g. those described in German patent applications nos. 1302847, 2020133 and 2065879 and British patent specifications no. 1327270 and 1347979.

However, the yields resulting from the use of such Dane salts appeared to be unsatisfactory as well for the purpose of the present invention, and moreover, these Dane salts appeared not to be available commercially.

Dutch patent application no. 6401841 further discloses the protection of the carboxylic group of 6-APA, 7-ACA and other amino acids by reacting it with dihalogensilane derivatives. These bi-functional silicon compounds are more easily accessible than the mono-functional trialkylhalogen-silanes and the application thereof does in a number of cases, lead to improved yields, as appears e.g. from British patent specification no. 1266544, disclosing the preparation of intermediate organosilane penicillins by reaction of 6-APA and these bi-functional silicon compounds. The organosilane derivatives are acylated to form, e.g. ampicillin. An expert, from account of the contents of this patent application, would have expected that the application of the organosilane penicillins described therein, would lead to interesting yields in the preparation of amoxicillin too. However, very surprisingly this expectation could not be confirmed by the results of initial experiments.

From later patent specifications, e.g. British patent specifications no. 1356737, 1404846 and 1459999 it is known to employ trivalent phosphorus derivatives instead of the above-mentioned silicon derivatives. A disadvantage of these phosphorus derivatives is that they cost 10 to 20 times more than the said silicon derivatives and this in combination with the toxicity and spontaneous inflammability of the di(lower alkyl)phosphorus derivatives as indicated in Inorganic Synthesis 15 (1974) pages 191—3, makes the phosphorus compounds a poor substitute for the silicon compounds.

Although it is further known from a number of patent specifications, e.g. Japanese patent applications no. 49-014687 and 49-048892, British patent specifications nos. 1367342 and 1382255 and German patent applications nos. 2460649 and 2621618, to prepare amoxicillin from 6-APA and p-hydroxyphenylglycine or lower alkyl esters thereof by enzymatic acylation, processes of this type are also unsatisfactory in view of the yields obtained and/or the presence of the acylating enzyme in the amoxicillin-containing solution obtained.

As a result of extensive research and development, an improved process for the preparation of 6-[D-$\alpha$-amino-p-(hydroxyphenyl)acetamido]penicillanic acid has surprisingly been found, characterized in that:

a) 6-aminopenicillanic acid is reacted in a dry, inert, water-immiscible main solvent with a trialkylsilyl group supplying silylating agent in an amount of approximately two equivalents with respect to the 6-aminopenicillanic acid, whereby possibly present amounts of free trialkylhalosilane, containing 1—3 carbon atoms in the alkyl residues, and of tertiary amine in the reaction mixture have been mutually balanced at the end of the reaction by adjusting to an empirically determined signal value,

b) the obtained reaction mixture, containing a derivative of 6-aminopenicillanic acid according to the formula:

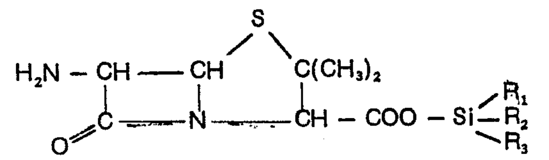

(I)

and/or

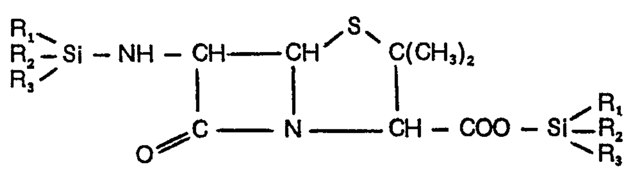

(II)

wherein each of $R_1$, $R_2$ and $R_3$ represents the same or different alkyl group of 1—3 carbon atoms and each preferably represents a methyl group, is cooled and subsequently is rapidly mixed under anhydrous conditions with a cooled solution of at least an equimolecular amount of a mixed anhydride

according to formula (III), which solution has been prepared by reacting the corresponding Dane salt with an alkyl chloroformate under anhydrous conditions in a water immiscible, inert, organic main solvent to which 0—25% by volume of an inert cosolvent has been added, whereafter

c) the acylation reaction is continued at a temperature below 0°C, followed by the recovery of the desired compound by methods known per se.

The reaction is preferably continued at —20°C to —30°C for 0.25 to 3 hours and preferably one to 2.5 hours and the reaction mixture is poured out into water while exactly establishing the pH at a value below 2.5. Preferably the pH is established at a value between 0.8 and 1.2.

The dry, inert, water-insoluble solvent for the silylating reaction is preferably dry methylene chloride and the silylating agent is preferably trimethylchlorosilane (TMCS), in the presence of a tertiary amine. However, good results may also be obtained with trimethylsilylacetamide, bis(trimethylsilyl)-acetamide, bis(trimethylsilyl)urea and hexamethyldisilazane.

The term "mutually balanced", as used under step (a) hereinbefore, can be clarified as indicated below.

It has been found, that optimal final yields can only be reached in a reproduceable manner by means of a very accurate balance between the amounts of agents employed to produce the silylated intermediate, especially in view of the desired optimal conversion with the prepared solution of the mixed Dane anhydride. It will be appreciated by persons skilled in the art, that — to point at just the main causes leading to undesirable deviation from the ideal balance — small errors in measuring out volumes or weighing out amounts are in practice inevitable, especially in experiments on very small scale (a few millimoles) or in production on large, industrial scale as well are impurities of technical reagents. What is needed then, is a "tool" whereby this necessary balance can be checked in situ and if necessary restored after completion of the silylation. The necessity and the advantage of "measuring this mutual balance" and its eventual restoration is a general feature of silylation procedures performed on 6-APA and the like, but is of preponderant importance when the silylation is carried out with the most economic agent combination, e.g. trimethylchlorosilane and triethyl amine, since such a combination — unlike single silylation agents like N,O-bistrimethylsilyl acetamide — does not provide for some sort of internal mutual balance by itself.

In the search for a suitable and at the same time simple method, by which this balance could be measured and if necessary corrected in situ, under technical conditions, it was surprisingly found, that for example the use of a good quality pH meter adequately fulfilled this demand. If for example a Radiometer pH meter type TTT2,C is employed in connection with a Radiometer GK 2401C electrode or an Ingold, so-called cold electrode at a temperature between 15 and 25°, a pH scale value in the empirical range between 5,5 and 7,5 and preferably between 6.0 and 7.2 should prevail at the end of the silylation reaction. If this is not the case a small additional amount of trimethylchlorosilane or e.g. triethylamine is added in order to effect an improved mutual balance.

It has appeared to be irrelevant whether or not the above indicated optimal pH scale interval does in fact correspond to an actual pH interval of the same order (6,0—7,2) to be measured after dissolution of the silylation mixture in water, since scale interval readings on other suitable pH-meters can be gauged to the one obtained with the indicated pH meter type. A feature of the present invention therefore is recognition of the need to control the mutual balance of agents in the silylation reaction as well as prescription of a simple method to solve this fundamental problem in situ.

The silylation is preferably carried out in dry dichloromethane employing approximately 2 equivalents of a tertiary amine as hydrochloric acid binding agent, such as triethylamine, and approximately 2 equivalents of a tri(lower)alkylhalogensilane, such as trimethylchlorosilane per mole of 6-aminopenicillanic acid under technical conditions.

The dry, water-insoluble main solvent used for the preparation of the so-called Dane anhydride may be dry methylene chloride to which dimethylformamide, sulfolane, tetrahydrofuran, N-methylpyrrolidone, 1,4-dioxane, acetonitrile, dimethylacetamide or tetramethylurea, or a mixture thereof, is added as a co-solvent to at most 25% by volume, or methylisobutylketone as main solvent to which one or more of the co-solvents mentioned above optionally may be added up to 25% by volume. Preferably potassium or sodium D-$\alpha$-(1-carbomethoxy-propen-2-yl)-amino-p-hydroxyphenyl-acetate is reacted with, preferably, methyl chloroformate in contrast with the opinions hitherto held to be true, as may be evident from Houben-Weyl, Methoden der Organischen Chemie, 4th Edition, (1974) Volume XV/2, Synthese von Peptiden, Part II, page 172, German patent applications nos. 2520647 and 2454841 and the Belgian patent no. 824158. N-methylmorpholine is preferably used as a catalyst. The chloroformate is preferably added to the Dane salt, while the reaction is preferably carried out at a temperature of —10°C or lower, preferably at a temperature between —10°C and —35°C. Mixtures of methylene chloride and the indicated co-solvents, with to about 20% by volume and preferably up to 10% by volume of co-solvent in the starting mixture, are proposed as the optimal solvents for the preparation of the Dane mixed anhydride.

Preferably tetrahydrofuran, N,N-dimethylacetamide, N,N-dimethylformamide, N-methylpyrrolidone and N,N,N',N'-tetramethylurea are used as co-solvents.

According to a further preferred process the solution of the anhydride is cooled to a temperature

of $-15°C$ or lower and a cooled solution of silylated 6-APA, is added rapidly with efficient stirring so that a temperature of $-15°C$ to $-30°C$ is reached, whereafter the reaction mixture is stirred for a further 0.5 to 3 hours. A small excess of the formula III compound is preferably employed.

The reaction mixture is then preferably mixed with a dilute solution of an inorganic acid such as a dilute (aqueous) hydrochloric acid solution, preferably in such a manner that the temperature becomes $-5°C$ to $+5°C$ and preferably around 0°C and the pH-value becomes 1.0 to 1.5. The mixture is stirred for a further 0.5 to 2 hours at the same temperature.

In a preferred procedure the layers are separated. The aqueous layer, containing the desired compound as its hydrochloride, is washed with an inert, water-insoluble, organic solvent, e.g. methyl isobutylketone or methylene chloride. The organic layer is washed with water and the washing is extracted with the organic washing. Then the washing of the aqueous layer is added to the washed aqueous layer. The aqueous layer is kept at a temperature of 0°C or lower, and the amoxicillin is recovered by crystallization in the usual way.

It will be appreciated that the initially isolated amoxicillin trihydrate may be further converted into non-toxic, pharmaceutically acceptable salts by methods known per se.

It will be appreciated that some of the most important advantages of the acylation process according to the invention are:

— previous and selective silylation of the p-hydroxy group of p-hydroxyphenylglycine is avoided

— the reaction is being carried in a concentrated solution of the reactants; given the size of the equipment this will favourably influence the output in kilos per batch

— the avoidance of the use of a p-hydroxyphenylglycine chloride hydrochloride which can only be prepared by a rather difficultly manageable process which moreover is rather expensive in a number of countries, due to very stringent safety regulations, if indeed permission for its manufacture can be obtained at all

— the use of large amounts of other additional chemicals is avoided

— the desired final product can surprisingly be prepared in economically attractive initial yields in a quality acceptable under existing health regulations while, at the same time, the number of purification steps can be reduced with attendant smaller losses of desired compound.

— mixing of the preferred water immiscible main solvent e.g. dichloromethane with a much smaller amount of a dipolar aprotic, water miscible solvent enables a simple and economically advantageous recovery of the main solvent.

— the chance of undesired and unexpected crystallization of 6-APA is practically nil, so that a reliable and rather trouble-free process is provided.

It will be appreciated that at least two undesired side reactions always will be considered by skilled people, when acylating by means of a mixed anhydride:

1) acylation, whereby alkoxycarbonylamino penicillanic acid derivatives will be formed;

2) partial racemisation of the N-protected amino acid, e.g. during the conversion of the N-protected amino acid or salt thereof into the mixed anhydride.

The avoidance of the first side reaction by rather simple means will be regarded as rather impossible by skilled people, relating to the fact that coupling of mixed anhydrides may take place at two reactive sides of the molecule and mixed anhydrides naturally are more or less labile and are inclined to disproportionate into two symmetrical anhydrides.

Hence the preparation of the mixed anhydride as well as the conversion of this anhydride with an amino acid will be always carried out at low temperatures.

The second undesired side reaction (racemisation) may be mainly avoided by trial and error methods.

Whereas according to the literature, ethylchloroformate and — in the event of racemisation when using this reactant, to a somewhat lesser extent — isobutylchloroformate, pivaloyl-chloride and benzoylchloride were regarded as suitable reactants as is known from e.g. German patent applications nos. 2520647 and 2454841 and the Belgian patent no. 824158, the use of methylchloroformate could not be recommended, it now appeared surprisingly that the use of the cheap methylchloroformate leads to significantly improved results.

It will be appreciated, that for the preparation of the mixed anhydride and the subsequent acylation, only solvent systems may be considered, which will meet the requirements of economy (recovery, recycling) and/or ecology.

These conditions can surprisingly be fulfilled by the certainly not predictable application of the presently proposed solvent systems.

The application of the solvent systems in the preparation of the mixed anhydride surprisingly appears to lead to:

a) an improved and moreover more reproduceable and reliable formation of the mixed anhydride and hence an improved conversion yield to the desired compound;

b) an improved yield of the desired compound in combination with a simultaneous increase of the concentrations to an attractive level, with reference to the present economical requirements;

c) the rather unexpected application of N,O-silylated 6-APA for the reaction with the mixed anhydrides in the indicated yields;

d) the possibility of carrying out both the silylation reaction and the preparation of the mixed anhydride in one and the same water immiscible main solvent, and more particularly dichloromethane, on account of which the presently required recovery of solvents is drastically simplified.

As in industrial processes the application of thorougly dried solvents and/or Dane salts of very high purity is an ideal that will never be realized completely, a slight excess of the starting Dane salts and of (lower)alkylchloroformates is preferably used.

In one of the preferred embodiments of the process of the present invention, amoxicillin trihydrate is prepared, starting from dry methylene chloride, 6-amino-penicillanic acid, trimethylchlorosilane in the presence of triethylamine in accurately balanced mutual amounts and in an amount of about two equivalents with reference to 6-APA, potassium D-$\alpha$-(1-carbomethoxypropen-2-yl)-amino-p-hydroxy-phenylacetate, N-methylmorpholine as catalyst, a cosolvent selected from N,N-dimethylacetamide, N-methylpyrrolidone, N,N-dimethylformamide, and tetramethylurea or mixtures thereof, and methyl chloroformate, while the solutions of the in situ prepared silylated 6-APA and the mixed anhydride are precooled to −40°C and the reaction is performed in two hours at a temperature of −30°C, followed by recovery of the amoxicillin by known methods.

An amoxicillin preparation prepared according to the described process is characterized by, e.g. the following analysis data:

| | |
|---|---|
| content measured by the hydroxylamine method (based on dry matter content) | 99.8% |
| content measured mercurometrically (based on dry matter content) | 98.7% |
| volatile components | 12.6% |
| gas-chromatographically measured contents: | |
| acetone | 15 mg/kg |
| methylene chloride | 119.5 mg/kg |
| methyl isobutylketone | 90 mg/kg |
| dimethylaniline | 2 mg/kg |
| $[\alpha]^{D}_{20}$ (on dry matter) | 301* |
| pH value | 5.0 |
| bulk density 6 taps: | 216 ml/100 g |
| 50 taps: | 204 ml/100 g |
| heavy metals | <10 ppm |
| sulphate ash | < 0.1% |
| germ number | < 10/g |
| decomposition products: penicilloinic acid | 0.4% |
| penicilloinic acid | 0.6% |
| solubility (clarity) HCl: | 0.6 EBC* |
| $NH_4OH$: | 0.5 EBC* |

*EBC = unity of clarity of a liquid according to the European Brewery Convention.

The following examples illustrate the process according to the invention without, however, being considered to be restrictive in any respect.

Example I

a) Preparation of methoxycarbonyl-D-$\alpha$-(1-carbomethoxy-propen-2-yl)-amino-p-hydroxyphenyl-acetate.

In a 2 l reaction vessel 58 g potassium D-$\alpha$-(1-carbomethoxypropen-2-yl)amino-p-hydroxyphenylacetate is weighed and 200 ml of methylene chloride dried previously by means of a molecular sieve A4 are added. After cooling the mixture to —40°C with stirring 25 ml of tetramethylurea are added, causing the temperature to rise to about —35°C. Then 0.5 ml of N-methylmorpholine and 16 ml of methylchloroformate are added subsequently. The temperature rises to about —30°C and the reaction mixture is stirred for 2 hours at —30°C at a pH-value of about 4. The reaction mixture is cooled to —40°C.

b) Silylation of 6-APA.

In a 1 l reaction vessel, 35 g of 6-aminopenicillanic acid are weighed and 350 ml of methylene chloride are added. With stirring 45 ml of triethylamine are added at room temperature and then 38 ml of trimethylchlorosilane (TMCS) are added in about 10 minutes, keeping the temperature at 20 to 25°C by cooling. After stirring for 1 hour at that temperature the pH is about 7.5.

By addition of 4 ml of TMCS the deflection on the scale of a Radiometer GK 2401C electrode, was brought to pH 6.0 $\pm$ 0.2.

c) Preparation of 6-[D-$\alpha$-amino-(p-hydroxyphenyl)acetamido]-penicillanic acid

The reaction mixture obtained according to b) is cooled to —40°C and is added at once to the solution of the mixed anhydride, causing the temperature to become —30°C. The reaction mixture is stirred at —30 to —25°C for 1 hour and is added to 800 ml of water, so that the temperature becomes 0°C and the pH becomes 2.5 to 3. The pH of the mixture is adjusted to 1.1 to 1.2 by the addition of 18 ml of concentrated hydrochloric acid. (36% by weight, chemically pure). After stirring at 0°C for 80 minutes the hydrolysis is complete.

The layers are separated and the aqueous layer is washed with 100 ml of methylene chloride. The organic layer is washed with 50 ml of distilled water and after extraction of the washing water with wash-methylene chloride the washing water is added to the aqueous layer. The coupling yield is about 92%, estimated on a sample. The aqueous layer is rapidly cooled to 0°C.

By crystallization the desired compound is obtained which, after filtration, is washed with 100 ml of 50% acetone-water and 100 ml of acetone and is dried in vacuo at about 30°C.

Yield about 55.5 g of amoxicillin trihydrate. Yield 82% of theoretical yield. The remaining mother liquor contains further about 10% of amoxicillin.

Example II

Using the same process as indicated in Example I, provided that 20 ml of dimethylacetamide is used instead of 25 ml tetramethylurea, a coupling yield of 92% was again obtained, and 55.5 g of pure amoxicillin trihydrate are obtained. Yield 82% of theoretical yield.

Example III

a,1) Preparation of ethoxycarbonyl D-$\alpha$-(1-carbomethoxypropen-2-yl)-amino-p-hydroxyphenylacetate

A thoroughly stirred suspension of 12.97 g (42.8 mmoles) of potassium d-$\alpha$-(1-carbomethoxy-propen-2-yl)-amino-p-hydroxyphenylacetate in 98 ml of methylisobutylketone (distilled over $K_2CO_3$) and 33 ml of tetrahydrofuran distilled over Redal, is cooled to —10°C under an atmosphere of dry $N_2$. Then 0.05 ml (5 drops from a Pasteur pipette) of N-methylmorpholine are added, followed by a solution of 4.2 ml (44 mmoles) of ethyl chloroformate, previously distilled and drawn under $N_2$, in 15 ml of methylisobutylketone. The reaction mixture is stirred at the same temperature for 30 minutes and the suspension is cooled to —20°C.

a,2) Preparation of methoxycarbonyl D-$\alpha$-(1-carbomethoxypropen-2-yl)-amino-p-hydroxyphenyl-acetate

A thoroughly stirred suspension of 45.6 mmoles of potassium D-$\alpha$-(1-carbomethoxypropen-2-yl)-amino-p-hydroxyphenylacetate in 98 ml of methylisobutylketone (distilled over $K_2CO_3$) and 33 ml of tetrahydrofuran distilled over Redal, is cooled to —10°C under an atmosphere of dry $N_2$. Then 0.05 ml of N-methylmorpholine are added, followed by a solution of 46.8 mmoles of methylchloroformate (purity 97%) in 15 ml of methylisobutylketone. The reaction mixture is stirred at the same temperature for 30 minutes and the suspension is cooled to —20°C.

b) Silylation of 6-APA.

In a nitrogen atmosphere 8.65 g (40 mmoles) of 6-APA are suspended in 130 ml of dry methylene chloride and 11.2 ml (80.6 mmoles) of triethylamine and 10.3 ml (82 mmoles) of trimethylchlorosilane are added subsequently. This mixture is refluxed with stirring for 1 hour and then cooled in an ice bath to below 5°C. The pH value measured with a Radiometer pH meter TTT2,C and a Radiometer GK 2401C electrode was adjusted to 6,4 at the end of the silylation reaction.

c,1) Preparation of 6-[D-α-amino-(p-hydroxyphenyl)acetamido]-penicillanic acid.

With vigorous stirring the turbid solution of the Dane anhydride, prepared under a,1) and cooled to −20°C, is added at once to a cooled solution of the silylated 6-APA obtained according to b), and the mixture is stirred for a further 20 minutes in an ice bath. Then the ice bath is removed but stirring is continued with introduction of nitrogen until room temperature is reached (about 45 minutes). The mixture is poured out , under cooling, in 75 ml of iced water, whereafter the pH, which reached a value of 2.5 to 3, is adapted to 1 to 1.2 with concentrated hydrochloric acid, as measured with an Electrofact KCl electrode or 0.5 to 0.7 with an AgCl electrode.

During the 30 minute's stirring with ice cooling a precipitate is not formed and, with ice cooling and stirring, a solution of 10% of KOH or NaOH is slowly added dropwise until the pH remains constant at 5.2 to 5.3 (in about 1 hour).

The reaction mixture is stored in a refrigerator for 20 hours and the precipitate formed is filtered off using a G3 glass filter. The product is thoroughly washed on the filter with water, followed by acetone.

The product is dried in vacuo (generated by an oil pump, about 1 mm Hg) over a siccapent for 16 to 24 hours. Amoxicillin trihydrate is obtained in a yield of 71.2% with a mercurometrically measured purity of 98.4% and a biologically measured quality of 96.9%.

c,2) Preparation of 6-[D-α-amino(p-hydroxyphenyl)acetamido]penicillanic acid.

In substantially the same manner as described hereinbefore under c,1) amoxicillin trihydrate is obtained in a yield of 83.5% having a purity of 96% according to hydroxylamine measurement, a biologically measured quality of 94.4% and an optical rotation $[\alpha]^{D}_{20}$ of +294°, starting from the solution of the Dane anhydride, prepared under a,2) instead of the solution prepared under a,1).

Example IV

In substantially the same manner as described under a,2), b) and c,2) in Example III amoxicillin trihydrate is obtained in a yield of 79.3%, having a purity of 97.6% according to hydroxylamine measurement, a biologically measured quality of 95.4% and an optical rotation $[\alpha]^{D}_{20}$ of +300°, starting from 45.6 mmoles of potassium D-α-(1-carbomethoxypropen-2-yl)-amino-p-hydroxyphenylacetate in 110 ml of methyl isobutylketone and 10 ml of N-methylpyrrolidone, 0.05 ml of N-methylmorpholine, 46.8 mmoles of methylchloroformiate (purity 97%) in 15 ml of methylisobutylketone, 40 mmoles of 6-APA in 130 ml of dry methylene chloride, 80.6 mmoles of triethylamine and 82 mmoles of trimethylchlorosilane.

The pH value measured with a Radiometer pH meter type TTT2,C and a Radiometer GK 2401C electrode was adjusted to 6.7 at the end of the silylation reaction.

Example V

a) Preparation of methoxycarbonyl D-α-(1-carbomethoxypropen-2-yl)amino-p-hydroxyphenylacetate.

In a 2 l reaction vessel 58 g of potassium D-α-(1-carbomethoxy-propen-2-yl)-amino-p-hydroxyphenylacetate are weighed and 400 ml of dry methyl isobutylketone are added. After cooling, with stirring, to −15°C 0.5 ml of N-methylmorpholine and 16 ml of methylchloroformate are added. The reaction mixture is stirred at −11°C for 1.5 hours and is then cooled to −43°C.

b) Silylation of 6-APA.

In a 1 l reaction vessel 35 g of 6-aminopenicillanic acid are weighed and 400 ml of methylene chloride are added. After addition of 73 g of bis(trimethylsilyl)urea the mixture is refluxed for about 2.5 hours. The mixture is cooled to 20°C.

The "pH" on the scale of a Radiometer pH meter type TTT2,C connected with a Radiometer GK 2401C electrode, reads 6.3.

c) After cooling the mixture according to b) is added as quick as possible to the cooled solution of the mixed anhydride, so that a temperature of −30°C is reached. The reaction mixture is stirred at −30 to −25°C for 1 hour and is added to 800 ml of water, so that the temperature becomes 0°C and the pH value becomes 2.5 to 3. The recovery is carried out in the same manner as described in Example I.

Yield 48.4 g of amoxicillin trihydrate having a biologically measured quality of 96.8%. Yield 71.3% of theoretical yield. The remaining mother liquor appears to contain a further about 10% of amoxicillin.

Example VI

In the same manner as described in Example V 48.3 g of amoxicillin trihydrate, having a purity of 97.7%, are obtained by reaction of methoxycarbonyl D-α-(1-carbomethoxypropen-2-yl)-amino-p-hydroxyphenylacetate and 35 g of 6-aminopenicillanic acid, previously silylated with 72.5 g of bis(trimethylsilyl)acetamide instead of the bis(trimethylsilyl)urea.

Example VII

a) Preparation of methoxycarbonyl D-α-(1-carbomethoxypropen-2-yl)-amino-p-hydroxyphenylacetate.

A thoroughly stirred suspension of 58 g of potassium D-α-(1-carbomethoxypropen-2-yl)amino-p-hydroxyphenylacetate in 400 ml of dry methyl isobutylketone (distilled over $K_2CO_3$) is cooled to −17°C under an atmosphere of dry nitrogen. Then 0.5 ml of N-methylmorpholine are added, followed by 16 ml of methylchloroformate. The temperature rises to −12°C and the reaction mixture is stirred at −12°C for 1.5 hours. The "pH" on the scale of a Radiometer pH meter, type TTT2,C, connected with a Radiometer GK 2401C electrode, finally reads 3.7. The reaction mixture is cooled to −40°C.

b) Silylation of 6-APA.

35 g of 6-APA are added to 400 ml of methylene chloride. After addition of 43 ml of triethylamine at ambient temperature and under stirring, 35 ml of trimethylchlorosilane are added in about 10 minutes at a temperature of 20—25°C. After additional stirring for 1 hour the "pH" value is adjusted to a final value of 6.7 by the addition of 4.9 ml of trimethylchlorosilan. The mixture is cooled to −40°C.

c) The cooled solution as obtained under b) is added as quick as possible to the cooled solution of the mixed anhydride as prepared under a), while the temperature rises to −30°C. The reaction mixture is stirred at −30°C for 1 hour and is subsequently added to 800 ml of water and 15 ml concentrated (36%) HCl solution, the temperature of which is 10°C. After additional stirring at 0°C the layers are separated and the aqueous layer is washed with 100 ml of methylene chloride. The organic layer is washed with 50 ml of distilled water and after extraction of the washing water with the wash methylene chloride the water layers are combined. The acylation yield appears to be about 89% according to a microbiological test on a sample. The aqueous layer is rapidly cooled to 0°C and a final pH of 5.2 is reached. The crystallised desired compound is washed with 100 ml of an acetone-water (1/1) mixture and 100 ml of acetone, and dried under vacuo at about 30°C.

The yield is 54.0 g of amoxicillin trihydrate having a mercurometrically measured purity of 98.7% and a purity of 99.8% measured by means of the hydroxylamine method, and an optical rotation $[\alpha]^D_{20}$ of +300° based on dry matter.

Yield is 80% of the theoretical yield.

The remaining mother liquor contains a further amount of 6—7% of amoxicillin.

Example VIII

In the same manner as described in Examples V—VII, 55.3 g of amoxicillin trihydrate are obtained in a yield of 81.5% having a purity of 99.8% according to a hydroxylamine method measurement, a biologically measured purity of 95% a mercurometrically measured purity of 98.6%, and an optical rotation $[\alpha]^D_{20}$ of +300°, starting from 58 g of potassium D-α-(1-carbomethoxypropen-2-yl)-amino-p-hydroxyphenylacetate in 200 ml of methylenechloride and 20 ml of N-methylpyrrolidone, 0.5 ml of N-methylmorpholine, 16 ml of methylchloroformate, 35 g of 6-APA in 200 ml of methylenechloride, 43 ml of triethylamine and 35 ml of trimethylchlorosilane. The "pH" value measured with a Radiometer pH meter TTT2,C and a Radiometer GK 2401C electrode was adjusted at 6.7 at the end of the silylation reaction, while the solution of the mixed anhydride as well as the solution of silylated 6-APA were both previously cooled to −40°C and reacted at −30°C for 2 hours (detected acylation yield 93,1%).

Example IX

In the same manner as described in Examples V—VII·56.1 g of amoxicillin trihydrate are obtained in a yield of 82,7% having a purity of 99.7% according to a hydroxylamine method measurement, a biologically measured purity of 95%, a mercurometrically measured purity of 98.7% and an optical rotation $[\alpha]^D_{20}$ of +300°, starting from 58 g of potassium D-α-(1-carbomethoxypropen-2-yl)-amino-p-hydroxyphenylacetate in 200 ml of methylenechloride and 20 ml of N,N-dimethylacetamide, 0.5 ml of N-methylmorpholine, 16 ml of methylchloroformate, 35 g of 6-APA in 200 ml of methylenechloride, 43 ml of triethylamine and 35 ml of trimethylchlorosilane. The "pH" value measured with a Radiometer pH meter TTT2,C and a Radiometer GK 2401C electrode was adjusted at 6.7 at the end of the silylation reaction, while the solutions of the mixed anhydride and of silylated 6-APA were precooled to −40°C and reacted at −30°C for 2 hours (detected acylation yield 95%).

Example X

In the same manner as described in examples V—VII amoxicillin was prepared in a 80% acylation yield starting from 58.0 g of potassium D-α-(1-carbomethoxypropen-2-yl)-amino-p-hydroxyphenylacetate in 400 ml of methylenechloride, 20 ml of N,N-dimethylformamide, 0.5 ml of N-methylmorpholine, 16 ml of methylchloroformate, 35 g of 6-APA in 200 ml of methylenechloride, 43 ml of triethylamine and 35 ml of trimethylchlorosilane. The "pH" value measured with the same equipment as in the preceeding examples was adjusted at 6.7 the end of the silylation reaction, while solutions of the mixed anhydride and of silylated 6-APA were precooled to −40°C and reacted at −30°C for 2 hours.

## Claims

1. Process for the preparation of 6-(D-$\alpha$-amino-p-hydroxyphenylacetamido)penicillanic acid by acylation of 6-aminopenicillanic acid in a water immiscible inert, organic main solvent with a solution of a mixed anhydride according to the general formula

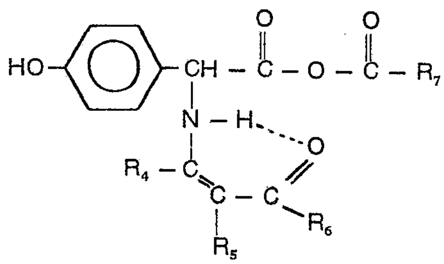

(III)

wherein $R_7$ represents an alkoxy group containing 1—2 carbon atoms, $R_6$ represents an alkoxy group containing 1—3 carbon atoms, $R_5$ represents an alkyl group containing 1—3 carbon atoms or a hydrogen atom, and $R_4$ represents an alkyl group containing 1—3 carbon atoms, which mixed anhydride solution has been previously prepared by reacting the corresponding Dane salt with an alkylchloroformate in an inert, organic main solvent and in the presence of a catalytic amount of a tertiary amine, characterised in that:

a) 6-aminopenicillanic acid is reacted in a dry, inert, water immiscible main solvent with a trialkylsilyl group supplying silylating agent in approximately 2 equivalents with respect to the 6-aminopenicillanic acid, whereby possibly present amounts of free trialkylhalosilane, containing 1—3 carbon atoms in the alkyl residues, and of tertiary amine in the reaction mixture have been mutually balanced by adjusting at the end of the reaction to an empirically determined signal value as defined in the specification,

b) the obtained reaction mixture, containing a derivative of 6-aminopenicillanic acid according to the formula:

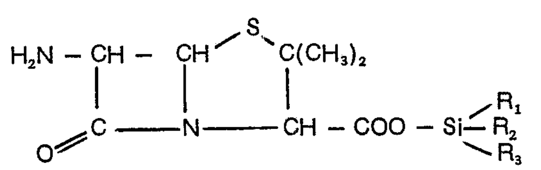

(I)

and/or

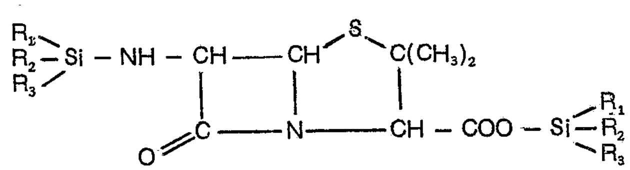

(II)

wherein each of $R_1$, $R_2$ and $R_3$ represents the same or different alkyl group of 1—3 carbon atoms and each preferably represents a methyl group, is cooled and subsequently is rapidly mixed under anhydrous conditions with a cooled solution of at least an equimolecular amount of a mixed anhydride according to formula (III), which solution has been prepared by reacting the corresponding Dane salt with an alkyl chloroformate under anhydrous conditions in a water immiscible, inert, organic main solvent to which 0—25% by volume of an inert cosolvent has been added, whereafter

c) the acylation reaction is continued at a temperature below 0°C, followed by the recovery of the desired compound by methods known per se.

2. Process according to claim 1, characterized in that 6-aminopenicillanic acid is reacted with trimethylchlorosilane in the presence of a tertiary amine, and in methylene chloride.

3. Process according to claim 2, characterized in that the possibly present amounts of free trimethylchlorosilane and of tertiary amine have mutually been accurately compensated so that the signal recorded by a pH electrode is adjusted at the end of the reaction at a constant value of a "pH scale value" between 5.5 and 7.5 at a temperature between 15—25°C, of a Radiometer pH meter type TTT2, and a Radiometer GK 2401C electrode or an Ingold cold electrode.

4. Process according to claims 1—3, characterized in that the mixed anhydride is previously prepared from the corresponding Dane salt and methyl chloro formate under anhydrous conditions, in

the presence of a tertiary amine as catalyst, for example N-methylmorpholine or N,N-dimethylbenzylamine, in dry methylene chloride mixed with a cosolvent or in methyl isobutyl ketone optionally mixed with a cosolvent.

5. Process according to claim 4, characterized in that the mixed anhydride is prepared in dry methylene chloride, to which dimethylformamide, sulfolane, tetrahydrofuran, N-methylpyrrolidone, 1.4-dioxane, acetonitrile, dimethylacetamide or tetramethylurea or a mixture thereof is added as a colsolvent, or in methyl isobutylketone as main solvent to which one or more of the said cosolvents may be added.

6. Process according to claim 5, characterized in that the solvent is dry methylene chloride, to which dimethylformamide, tetrahydrofuran, N-methylpyrrolidone, dimethylacetamide or tetramethylurea or a mixture thereof is added.

7. Process according to claim 6, characterized in that the cosolvent is added in an amount of up to 10% by volume.

8. Process according to any one of claims 1—7, characterized in that the preparation of the Dane mixed anhydride is carried out at a temperature of −10°C to −35°C.

9. Process for preparing the Dane mixed anhydride according to any one of claims 1—8, characterized in that sodium or potassium D-α-(1-carbomethoxypropen-2-yl)-amino-p-hydroxyphenylacetate is reacted with methylchloroformate.

## Patentansprüche

1. Verfahren zur Herstellung von 6-(D-α-Amino-p-hydroxyphenylacetamido)penicillansäure durch Acylieren von 6-Aminopenicillansäure in einem mit Wasser nicht mischbaren inerten organischen Hauptlösungsmittel, mit einer Lösung eines gemischten Anhydrids der allgemeinen Formel III

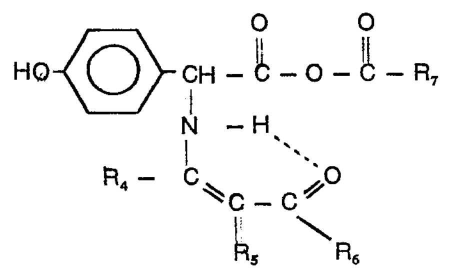

(III),

worin $R_7$ eine Alkoxygruppe mit 1 bis 2 Kohlenstoffatomen, $R_6$ eine Alkoxygruppe mit 1 bis 3 Kohlenstoffatomen, $R_5$ eine Alkylgruppe mit 1 bis 3 Kohlenstoffatomen oder ein Wasserstoffatom und $R_4$ eine Alkylgruppe mit 1 bis 3 Kohlenstoffatomen bedeuten, wobei die gemischte Anhydridlösung vorgängig durch Umsetzung des entsprechenden Danesalzes mit einem Alkylchlorformiat in einem inerten organischen Hauptlösungsmittel und in Gegenwart einer katalytischen Menge eines 3-wertigen Amins hergestellt wurde, dadurch gekennzeichnet, dass:

a) 6-Aminopenicillansäure in einem trockenen inerten, mit Wasser nicht mischbaren Hauptlösungsmittel mit 2 Aequivalenten, bezogen auf die 6-Aminopenicillansäure, eines eine Trialkylsilylgruppe abgebenden Silylierungsmittels umsetzt, wobei gegebenenfalls vorhandene Mengen von freiem Trialkylhalogensilan mit 1 bis 3 Kohlenstoffatomen in den Alkylresten und von tertiärem Amin im Reaktionsgemisch, gegenseitig ausgeglichen wurden durch Einstellen am Ende der Reaktion auf einen empirisch festgestellten Signalwert, wie in der Beschreibung angegeben,

b) das erhaltene Reaktionsgemisch, enthaltend ein Derivat der 6-Aminopenicillansäure der Formel I und/oder II

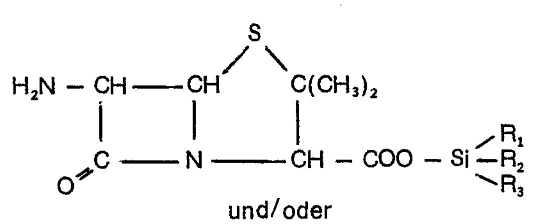

(I)

und/oder

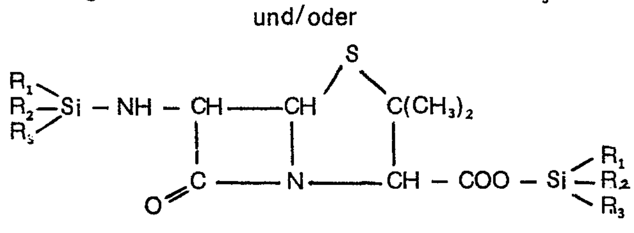

(II),

worin $R_1$, $R_2$ und $R_3$ gleiche oder verschiedene Alkylgruppen mit 1 bis 3 Kohlenstoffatomen und jedes bevorzugt eine Methylgruppe bedeutet, gekühlt und danach schnell unter wasserfreien Bedingungen mit einer gekühlten Lösung von mindestens einer äquimolaren Menge eines gemischten Anhydrids gemäss Formel III vermischt wird, wobei die Lösung durch Umsetzung des entsprechenden Danesalzes mit einem Alkylchlorformiat unter wasserfreien Bedingungen in einem mit Wasser nicht mischbaren inerten organischen Hauptlösungsmittel, zu dem 0 bis 25 Vol.-% eines inerten Colösungsmittels gegeben wurden, hergestellt wurde, wonach

c) die Acylierung bei einer Temperatur unterhalb 0°C, gefolgt durch die Gewinnung der gewünschten Verbindung nach bekannten Methoden, fortgesetzt wird.

2. Verfahren nach Anspruch 1, dadurch gekennzeichnet, dass 6-Aminopenicillansäure mit Trimethylchlorsilan in Gegenwart eines tertiären Amins und in Methylenchlorid umgesetzt wird.

3. Verfahren nach Anspruch 2, dadurch gekennzeichnet, dass gegebenenfalls vorhandene Mengen von freiem Trimethylchlorsilan und tertiärem Amin gegenseitig genau kompensiert wurden, derart, dass das durch eine pH-Elektrode registrierte Signal am Ende der Reaktion auf einen konstanten Wert auf einer pH-Wertskala zwischen 5,5 und 7,5 bei einer Temperatur zwischen 15 und 25°C auf einem Radiometer pH-Meter TTT2 und mit einem Radiometer GK 2401C-Elektrode oder einer Ingold-Kaltelektrode eingestellt wurden.

4. Verfahren nach den Ansprüchen 1 bis 3, dadurch gekennzeichnet, dass das gemischte Anhydrid vorgängig aus dem entsprechenden Danesalz und Methylchlorformiat unter wasserfreien Bedingungen in Gegenwart eines tertiären Amins als Katalysator, beispielsweise von N-Methylmorpholin oder N,N-Dimethylbenzylamin, in trockenem, mit einem Colösungsmittel gemischten Methylenchlorid oder in gegebenenfalls mit einem Colösungsmittel gemischten Methylisobutylketon, hergestellt wird.

5. Verfahren nach Anspruch 4, dadurch gekennzeichnet, dass das gemischte Anhydrid in trockenem Methylenchlorid zu dem Dimethylformamid, Sulfolan, Tetrahydrofuran, N-Methylpyrrolidon, 1,4-Dioxan, Acetonitril, Dimethylacetamid oder Tetramethylharnstoff oder deren Gemisch als Colösungsmittel gegeben wird, oder in Methylisobutylketon als Hauptlösungsmittel zu dem eine oder mehrere der genannten Colösungsmittel gegeben werden können.

6. Verfahren nach Anspruch 5, dadurch gekennzeichnet, dass das Lösungsmittel trockenes Methylenchlorid ist, zu dem Dimethylformamid, Tetrahydrofuran, N-Methylpyrrolidion, Dimethylacetamid oder Tetramethylharnstoff oder ein Gemisch davon, gegeben wird.

7. Verfahren nach Anspruch 6, dadurch gekennzeichnet, dass das Colösungsmittel in einer Menge von bis zu 10 Vol.-% zugegeben wird.

8. Verfahren nach einem der Ansprüche 1 bis 7, dadurch gekennzeichnet, dass die Herstellung des Dane gemischten Anhydrids bei einer Temperatur von —10 bis —35°C ausgeführt wird.

9. Verfahren zur Herstellung des Dane gemischten Anhydrids nach einem der Ansprüche 1 bis 8, dadurch gekennzeichnet, dass Natrium- oder Kalium-D-$\alpha$-(1-carbomethoxypropen-2-yl)-amino-p-hydroxyphenylacetat mit Methylchlorformiat umgesetzt wird.

## Revendications

1. Procédé de préparation de l'acide 6[D-$\alpha$-amino-p-hydroxyphénylacétamido]pénicillanique par acylation d'acide 6-aminopénicillanique dans un solvant principal organique inerte non miscible à l'eau au moyen d'une solution d'un anhydride mixte de formule générale:

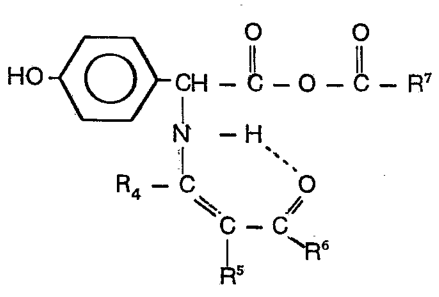

(III)

où $R^7$ représente un radical alkoxy contenant 1 ou 2 atomes de carbone, $R^6$ représente un radical alkoxy contenant 1 à 3 atomes de carbone, $R^5$ représente un radical alkyle contenant 1 à 3 atomes de carbone ou un atome d'hydrogène et $R^4$ représente un radical alkyle contenant 1 à 3 atomes de carbone, laquelle solution d'anhydride mixte a été préparée auy préalable par réaction du sel de Dane correspondant avec un chloroformiate d'alkyle dans un solvant principal organique inerte et en présence d'une quantité catalytique d'une amine tertiaire, caractérisé en ce que:

a) on fait réagir l'acide 6-aminopénicillanique, dans un solvant principal non miscible à l'eau inerte sec, avec un agent de silylation apportant un radical trialkylsilyle en quantité d'environ 2 équivalents par rapport à l'acide 6-aminopénicillanique, tandis que les quantités éventuellement présentes de trialkylhalogénosilane libre contenant 1 à 3 atomes de carbone dans les radicaux alkyle et d'amine tertiaire dans le mélange de réaction ont été mutuellement équilibrées par ajustement au terme de la réaction jusqu'à une valeur de consigne déterminée empiriquement comme défini dans le mémoire,

b) on refroidit le mélange de réaction résultant contenant un dérivé d'acide 6-aminopénicillanique de formule:

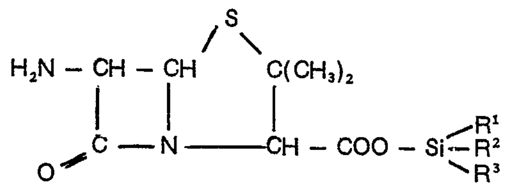

(I)

et/ou

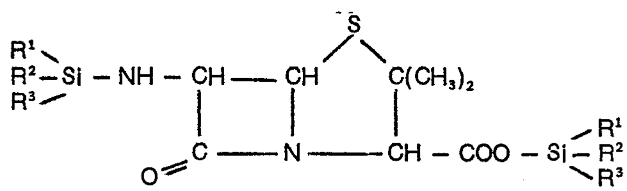

(II)

où chacun d'entre $R^1$, $R^2$ et $R^3$ représente un radical alkyle identique ou différent de 1 à 3 atomes de carbone et représente de préférence un radical méthyle, puis ou le mélange rapidement, dans des conditions anhydres, avec une solution refroidie d'au moins une quantité équimoléculaire d'un anhydride mixte de formule (III), laquelle solution a été préparée par réaction du sel de Dane correspondant avec un chloroformiate d'alkyle, dans des conditions anhydres, dans un solvant principal organique inerte non miscible à l'eau qui a été additionné de 0 à 25% en volume d'un cosolvant inerte, après quoi

c) on poursuit la réaction d'acylation à une température inférieure à 0°C, puis en recueille le composé désiré suivant des techniques connues.

2. Procédé suivant la revendication 1, caractérisé en ce qu'on fait réagir l'acide 6-aminopénicillanique avec le triméthylchlorosilane en présence d'une amine tertiaire et dans le chlorure de méthylène.

3. Procédé suivant la revendication 2, caractérisé en ce que les quantités éventuellement présentes de triméthylchlorosilane libre et d'amine tertiaire ont été compensées mutuellement avec précision de manière que le signal enregistré par une électrode de pH soit ajusté au terme de la réaction à une valeur constante "d'une valeur d'échelle de pH" entre 5,5 et 7,5 à une température de 15 à 25°C sur un pHmètre Radiometer type TTT2 avec une électrode GK 2401C Radiometer ou une électrode froide Ingold.

4. Procédé suivant les revendications 1—3, caractérisé en ce que l'anhydride mixte est préparé au préalable à partir du sel de Dane correspondant et de chloroformiate de méthyle, dans des conditions anhydres, en présence d'une amine tertiaire comme catalyseur, par exemple la N-méthylmorpholine ou la N,N-diméthylbenzylamine, dans du chlorure de méthylène sec mélangé avec un cosolvant ou dans de la méthylisobutylcétone éventuellement mélangée avec un cosolvant.

5. Procédé suivant la revendication 4, caractérisé en ce que l'anhydride mixte est préparé dans du chlorure de méthylène sec auquel du diméthylformamide, du sulfolane, du tétrahydrofuranne, de la N-méthylpyrrolidone, du 1,4-dioxanne, de l'acétonitrile, du diméthylacétamide ou de la tétraméthylurée ou bien un mélange de ces composés est ajouté comme cosolvant ou bien dans de la méthylisobutylcétone comme solvant principal auquel un ou plusieurs de ces cosolvants peuvent être ajoutés.

6. Procédé suivant la revendication 5, caractérisé en ce que le solvant est le chlorure de méthylène sec auquel du diméthylformamide, du tétrahydrofuranne, de la N-méthylpyrrolidone, du diméthylacétamide ou de la tétraméthylurée ou bien un mélange de ces composés est ajouté.

7. Procédé suivant la revendication 6, caractérisé en ce que le cosolvant est ajouté en quantité s'élevant jusqu'à 10% en volume.

8. Procédé suivant l'une quelconque des revendications 1 à 7, caractérisé en ce que la préparation de l'anhydride mixte Dane est exécutée à une température de —10 à —35°C.

9. Procédé de préparation de l'anhydride mixte de Dane suivant l'une quelconque des revendications 1 à 8, caractérisé en ce qu'on fait réagir le D-α[1-carbométhoxypropén-2-yl]-amino-p-hydroxyphénylacétate de sodium ou de potassium avec le chloroformiate de méthyle.